# EUROPEAN PATENT APPLICATION

(11) **EP 2 116 209 A1**
(43) Date of publication of application: **11.11.2009**
(21) Application number: 07834957.8
(22) Date of filing: 31.07.2007
(51) Int. Cl.: A61B 19/00, G02B 21/00

(54) **SURGERY MICROSCOPE CONTROL DEVICE**

(30) Priority: 07.03.2007 RU 2007108463
(71) Applicant: Pitskhelauri, David Ilich, Moscow 109263 (RU)
(72) Inventor: PITSKHELAURI, David Ilich, Moscow, 109263 (RU); KONOVALOV, Aleksander Nikolaevich, Moscow, 127055 (RU); SHEKUTIEV, Georgy Aleksandrovich, Moscow, 125047 (RU); RASSOKHIN, Anatoly Egorovich, Moscow, 121596 (RU); INAURI, Georgy Alekseevich, Moscow, 123376 (RU)
(74) Representative: HOFFMANN EITLE
(86) International application number: PCT/RU2007/000410
(87) International publication number: WO 2008/108678

(57) **Abstract**

The invention relates to medical equipment, in particular to devices for controlling a surgery microscope that can be used during a surgery operation. The inventive surgery microscope control device is embodied in the form of a bent holder (1) which is arranged along a surgeon head from the parietooccipital area through the facial area to a chin area. It is provided with means for fixing to the microscope eyepieces (2) and with supports (7, 8, 10, 12) for interacting with the surgeon's head in the bridge of nose, chin, frontal and parietooccipital areas; the support (8) interacting with the chin being provided with an electric switch (9). Optionally, a joystick (26) is mounted on the holder (1). Said device makes it possible to duplicate the controls of the microscope and not to divert the surgeon hands from surgery for controlling the microscope. The surgery microscope is controlled by means of the head, mouth (lips) and chin of the surgeon.

## Description

### Field of the invention

The invention relates to medical equipment, in particular to devices for controlling a surgery microscope. It can be used during surgical operations.

### Background of the invention

Binocular microscopes are traditionally used for surgical operations. For example OPMI Neuro/NC4 (manufactured by Karl Zeiss; System Neuro 200, Gebrauchsanweisung, G 30-1275-de, Ausgabe 1.0, Gedruckt am 08.01.1999, Zeiss; *http*//*www.zeiss.de, http*/*www.zeiss.com*/*neuron)* has two functional handles duplicating each other and having keys for controlling the microscope. A surgeon controls the microscope by hands during the surgical operation, i.e., the surgeon must interrupt an operation for moving a hand or hands of both arms to the hand grip or hand grips for controlling the microscope. Such arrangement does not provide for any change in the position of the microscope and its attachments in relation to a operative field (positioning, focusing and zoom adjustment etc.) while the surgeon's hands are busy with the operations.

In view of above it is clear that there is a need for a hand-free control system for a surgery microscope.

One of the examples of solving this problem in the prior art is a system for controlling a surgery microscope disclosed in European patent EP 1738710 (published on 03.01.2007). The system is the closest reference to the claimed invention. The known system comprises a commutator used by a surgeon for controlling a surgery microscope by his mouth not interrupting hand manipulations in the operation area.

The disadvantage of the system is limited scope of control parameters. Controlling by mouth is in fact comprises only switching on and off the commutating means. The ability to change positions of the microscope in relation to the object (axial movements and especially turns around axes) using the commutating means clamped by surgeon's teeth is rather limited.

### Summary of the invention

The objective of the present invention is a multifunctional device for controlling a surgery microscope with the help of surgeon's head, mouth (lips) and chin.

The device for controlling a surgery microscope according to the present invention comprises a bend holder placed around the surgeon's head from its parietooccipital area through the face area to the area under the chin. The holder is provided with a means of securing it on the microscopic eyepieces, supports on the surgeon's head in the areas of the bridge of nose, chin, forehead and back of head. The support on the chin is provided with an electric switch.

Preferably there is an electric connector coupled to the switch for connecting the device to the microscope.

It is advisable to locate the supports at the ends of the holder brackets on the surgeon's head at both sides in the forehead and back of the head areas. For adjusting the device to a particular head size, the brackets are designed adjustable and fixable in certain positions along the holder.

The brackets are bent to fit a head tightly.

The adjustable supports provide for more accurate fitting the surgeon's head.

The holder can be provided with a hinge and a lock between the brackets for fixing or releasing the holder on the surgeon's head.

Preferably the lock is equipped with an arm.

It is also desirable to use a detachable extender for the arm to facilitate sterilization of the device elements being in contact with the surgeon's hands.

The support for contacting the chin is adjustable and fixable relative to the holder.

The electric switch is used for sending a control signal to the blocking device of the microscope hinged bearings.

The device can be optionally provided with a joystick attached to the holder and designed to be in contact with surgeon's lips.

The joystick is used for directing signals to the focusing mechanism and/or zooming mechanism of the microscope objective.

The joystick can be electrically connected to the electric connector attached to the holder, said connecter being used for both the joystick and the electric switch.

In one of the device embodiments a means for fixing the holder on the microscope eyepieces is in the shape of a frame embracing the eyepieces, wherein the support for the bridge of nose attached to the holder between said eyepieces can be adjusted depending on personal form and size of the surgeon's bridge of nose.

Optionally the holder is designed adjustable in size and distance to the chin and/or the forehead area of the surgeon above and/or below the support for the bridge of nose.

### Brief Description of Drawings

The invention is further described with references to the following drawings:
Fig. 1 is a general view of the device for controlling a microscope;
Fig. 2 is a top view of a device portion with brackets of the supports placed on a surgeon's head in the forehead and parietooccipital areas;
Fig. 3 is a side view of one of the brackets with a support;
Fig. 4 is a part of the holder with a hinge and a lock;
Fig. 5 is a disassembled view of a means for placing the holder on microscope eyepieces;
Fig. 6 is a view of a variant of the device for controlling a microscope.

### Description of the Intention

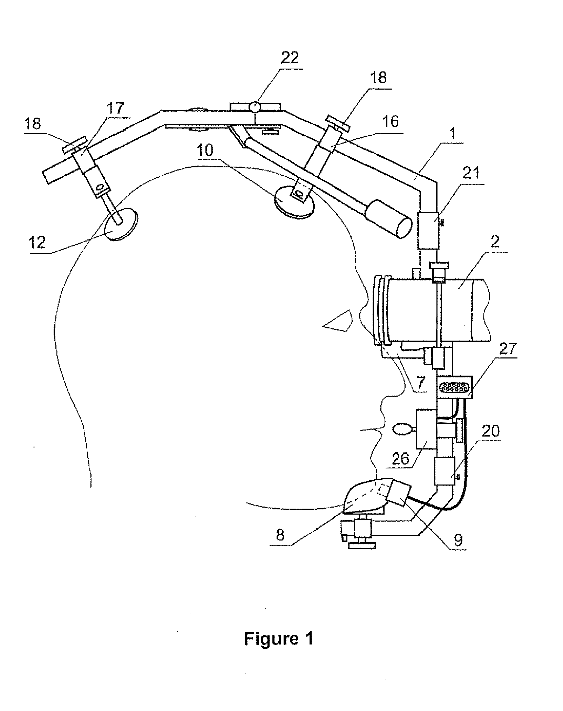

As shown in Fig. 1, the main element of the device controlling a surgery microscope is an arched holder 1. The holder 1 is arced to fit the profile of a head from the parietooccipital area through the face area to the area under the chin. The holder 1 is made of an aluminium alloy as the majority of other parts of the device. The cross-section of the holder 1 has the shape of a square or a rectangle.

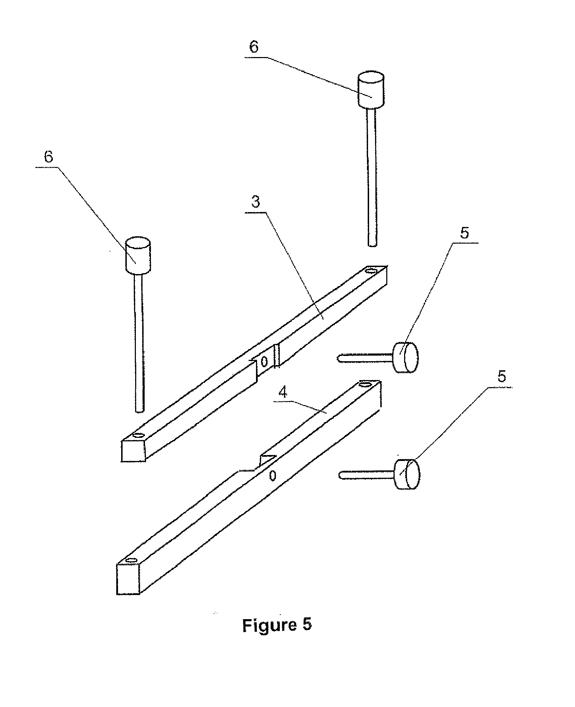

The holder 1 is provided with a means for placing it on the eyepieces 2 of the microscope. A variant of the means is shown in Fig. 5. It comprises a frame embracing the eyepieces 2. Cross beams 3, 4 of the frame are attached to the holder 1 with screws 5. The holder 1 is fixed to the eyepieces with screws 6.

A support 7 for resting on the bridge of nose is attached to the holder between said eyepieces so that its position can be adjusted depending on individual shape and size of a surgeon's bridge of nose.

The shape of the support 7 corresponds to the shape of the bridge of nose.

A support 8 for the chin area has a shape of the chin, and it can be adjusted and fixed in relation to the holder 1. The support 8 is provided with an electric switch 9 for transmitting control signals to the blocking mechanism of the microscope pivoting support.

Supports 10, 11 and 12, 13 contacting the surgeon's head in the forehead and parietooccipital areas are located at the ends of corresponding brackets 14 and 15.

For adjusting the device to fit a particular head, the brackets 14 and 15 are attached to the holder so that they can be adjusted and fixed in a certain position along the holder 1 using guiding elements 16 and 17 and screws 18.

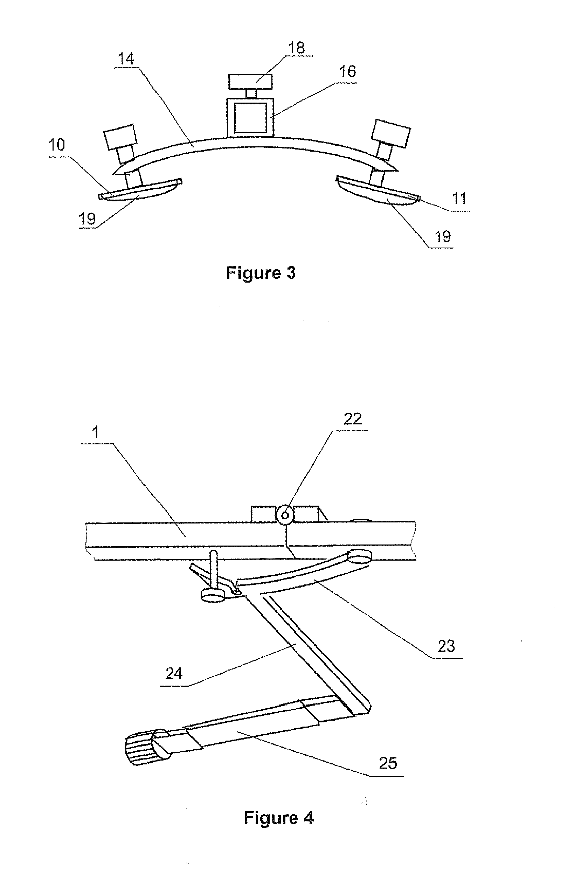

As shown in Fig. 3, the brackets 14 and 15 are arced for tighter contacts to the surgeon's head.

The supports 10, 11, 12, and 13 are adjustable upwards relative to the brackets 14 and 15 for tighter fitting the surgeon's head.

The surfaces of the supports 10, 11, 12, and 13 facing the surgeon's head have glued soft linings 19.

Optionally the holder 1 is designed adjustable in size and shape in the portion above or lower of the support 7 for the bridge of nose. Adjusters 20 and 21 are provided for the purpose.

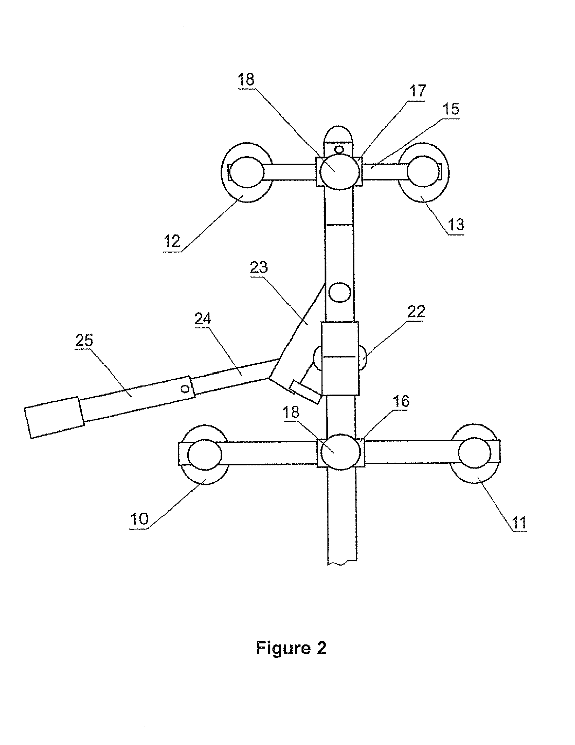

In the zone between the brackets 14 and 15 (Fig. 2 and Fig. 4) the holder 1 has a hinge 22 and a lock 23 for fixing or releasing the surgeon's head by raising the part of the holder 1 with the bracket 15.

For the sake of convenience the lock 23 is provided with an arm 24.

It is preferable to use detachable extender 25 for the arm 24 to facilitate sterilization of the device elements being in contact with the surgeon's hands.

For widening the functionality, the device can be optionally provided with a joystick 26 attached to the holder 1 and designed to be in contact with surgeon's lips. The joystick 26 is used for transmitting signals to the objective focusing mechanism and/or zooming mechanism of the microscope objective.

An electric connector 27 is attached to the holder 1 for connecting the device to the microscope control system. The electric switch 9 and the joystick 27 are connected to the connector 27. Such arrangement allows duplicating controls located on the microscope.

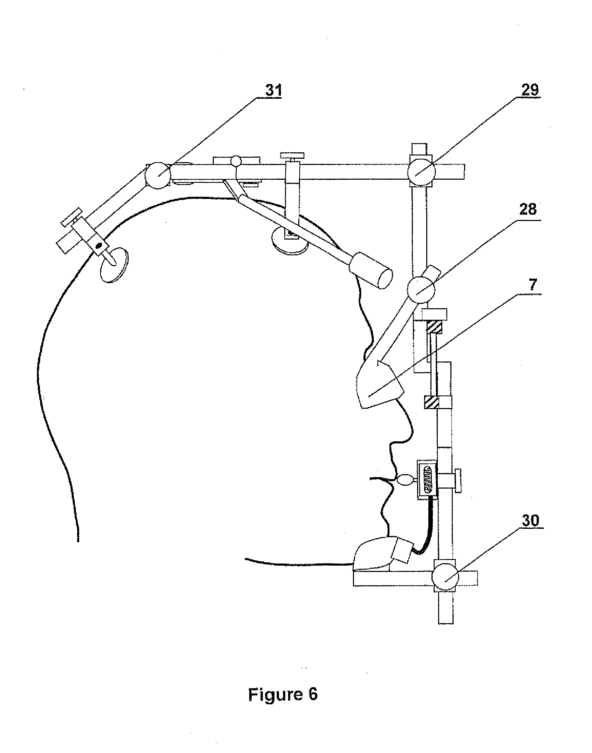

Alternative embodiment of the device is shown in Fig. 6. The adjustable nose support 7 is fixed to the holder by hinge 28. Adjusters 29 and 30 are used for adjusting the holder sizes along two axes. Hinge 31 allows changing inclination of the support interacting with the parietooccipital area of the surgeon's head.

Preparation of the device for controlling a microscope and further operations is conducted as follows.

The device is attached to the eyepieces 2 of the microscope by the means for fixing the holder 1. The device is electrically connected to the microscope through a multiconductor cable and the electrical connector 27. Then the microscope is balanced. The device is attached to the surgeon's head and adjusted by manipulating the supports (in the head, bridge of nose and chin areas), brackets and length of elements so that the surgeon feels only slight contacts with the supports without any pressure on the head. A sterilized detachable extender 25 is placed on the arm 24.

The rear part of the holder 1 is raised in the upper position turning around the hinge 22 with the help of the extender 25 of the arm 24, and the surgeon's head is positioned in relation to the microscope eyepieces 2 and the bridge-of-nose support 7. Then the rear part of the holder 1 is lowered to the parietooccipital area of the surgeon's head with the help of the extender 25 of the arm 24 and fixed with the lock 23.

Relatively rigid attachment of the device for controlling a microscope on a surgeon's head and simultaneous unblocking of the microscope hinged bearings is initiated when the mouth is open and the chin is slightly moved ahead. This manipulation brings two results: 1) a tight adjustment to the surgeon's head and in general to the microscope is achieved (when the mouth is open, the head as though being "stuck" between the supports of the holder upper part and the chin and nose); 2) the electric switch 9 is pressed causing unblocking of the microscope hinged bearings. As the result, the surgeon can direct the microscope at any place (along X, Y and Z axes as well as around them). After a required position is achieved, the surgeon closes his mouth returning the electrical switch in off position and blocking the microscope hinged bearings. At the same time the pressure of the device supports on the surgeon's head is released. Then the surgeon makes surgical manipulations until the next step of moving the microscope as described above.

For initiating control signals (objective focusing, zooming) the surgeon moves his/her lips slightly ahead to the joystick 26 arm. When the lips are moved slightly up, down, left or right, corresponding signals (focus-up, focus-down, zoom-in, zoom-out) are transmitted to the microscope control system.

The embodiment of the invention described above does not limit the scope of the invention. It is obvious to a person skilled in the art that many elements can have different structures within the frames of the present invention and that the scope of what to be protected is defined only by the claims of the invention. It is also obvious that the device can be used for controlling different types of microscopes.

## Claims

1. A surgery microscope control device in the form of a bent holder arranged above a surgeon's head from its parietooccipital area through the face area to the area under the chin, the holder being provided with a means of securing it on the microscopic eyepieces, supports on the surgeon's head in the bridge of nose, chin, forehead and parietooccipital areas, wherein the support on the chin is provided with an electric switch.

2. The device according to claim 1 **characterized in that** an electric connector coupled to said switch is attached to the holder.

3. The device according to claim 1 **characterized in that** the supports placed on the surgeon's head in the forehead as well as in the parietooccipital area are located at both sides in relation to the holder at the ends of the brackets mounted on the holder.

4. The device according to claim 3 **characterized in that** the brackets are designed adjustable and fixable in certain positions along the holder.

5. The device according to claim 3 **characterized in that** the brackets are bent brackets.

6. The device according to claim 1 **characterized in that** the supports are adjustable supports.

7. The device according to claim 1 **characterized in that** a hinge and a lock are mounted in the zone between the brackets for fixing or releasing the holder on the surgeon's head.

8. The device according to claim 7 **characterized in that** the lock is provided with an arm.

9. The device according to claim 8 **characterized in that** said arm is provided with an extender.

10. The device according to claim 1 **characterized in that** the support for the chin is arranged so that its position is adjustable and fixable relative to the holder.

11. The device according to claim 1 **characterized in that** the electric switch is used for directing a control signal to the blocking device of the microscope hinged bearings.

12. The device according to claim 1 **characterized in that** said device is provided with a joystick attached to the holder and designed to interact with surgeon's lips.

13. The device according to claim 12 **characterized in that** the joystick is connected to the electrical connector attached to the holder.

14. The device according to claim 12 **characterized in that** the joystick is designed for directing signals to the focusing mechanism and/or zooming mechanism of the microscope objective.

15. The device according to claim 12 **characterized in that** the means for fixing the holder on the microscope eyepieces is in the form of a frame embracing the eyepieces, wherein the support for the bridge of nose attached to the holder between said eyepieces can be adjusted depending on personal form and size of the surgeon's bridge of nose.

16. The device according to claim 1 **characterized in that** the holder is designed adjustable in size and shape in the portion being above and/or below the support for the bridge of nose.
